# EUROPEAN PATENT SPECIFICATION

(11) **EP 0 625 912 B1**
(45) Date of publication and mention of the grant of the patent: **16.04.1997**
(21) Application number: 93904830.2
(22) Date of filing: 02.02.1993
(51) Int. Cl.: A61K 39/395

(54) **TREATMENT FOR INFLAMMATORY BOWEL DISEASE**
BEHANDLUNG FÜR ENTZÜNDUNGSERKRANKUNG DES DARMES
TRAITEMENT DE L'INFLAMMATION INTESTINALE

(30) Priority: 12.02.1992 US 835139
(43) Date of publication of application: 30.11.1994
(73) Proprietor: BIOGEN, INC., Cambridge Massachusetts 02142 (US)
(72) Inventor: LOBB, Roy, R., Westwood, MA 02090 (US)
(74) Representative: Ruffles, Graham Keith
(86) International application number: US9300924
(87) International publication number: WO9315764

(56) References cited:
- EP-A- 0 314 863
- EP-A- 0 346 078
- WO-A-92/00751
- WO-A-93/13798
- PROCEEDINGS OF THE NATL. ACADEMY OF SCIENCES USA, vol. 88, no. 16, 15 August 1991, Washington, DC (US); P.F. WELLER et al., pp. 7430-7433/
- GASTROENTEROLOGY, vol. 103, no. 3, September 1992, New York, NY (US); M. KOIZUMI et al., pp. 840-847/
- J Biol Chem vol. 266/16, 1991, pp.10241-10245
- J Clin Invest vol. 92, July 1993, pp. 372-380

## Description

### FIELD OF THE INVENTION

The present invention relates to a treatment for inflammatory bowel disease (IBD). More particularly, this invention relates to the use of antibodies recognizing the integrin VLA-4 (very late antigen-4) in the treatment of IBD.

### BACKGROUND OF THE INVENTION

Inflammatory bowel disease, or IBD, is a collective term encompassing ulcerative colitis and Crohn's disease (ileitis), which are chronic inflammatory disorders of the gastrointestinal tract. Ulcerative colitis is confined to the large intestine (colon) and rectum, and involves only the inner lining of the intestinal wall. Crohn's disease may affect any section of the gastrointestinal tract (i.e., mouth, esophagus, stomach, small intestine, large intestine, rectum and anus) and may involve all layers of the intestinal wall. Both diseases are characterized by abdominal pain and cramping, diarrhea, rectal bleeding and fever. The symptoms of these diseases are usually progressive, and sufferers typically experience periods of remission followed by severe flareups.

IBD affects an estimated two million people in the United States alone. Although IBD is not considered a fatal illness, prolonged disease can lead to severe malnutrition affecting growth or to the formation of abscesses or intestinal scar tissue, leading in turn to infection or bowel obstruction.

IBD has no cure, and the exact causes of IBD are not yet understood. Conventional treatments for IBD have involved anti-inflammatory drugs, immunosuppressive drugs and surgery. Sulfasalazine and related drugs having the bioactive 5-amino-salicylic acid (5-ASA) moiety are widely used to control moderate IBD symptoms and to maintain remission. Severe inflammation is often treated with powerful corticosteroids and sometimes ACTH or with immunosuppressants such as 6-mercaptopurine and azathioprine. The most common surgical treatments for severe chronic IBD are intestinal resections and, ultimately, colectomy, which is a complete cure only for ulcerative colitis.

Severe side effects are associated with the drugs commonly prescribed for IBD, including nausea, dizziness, changes in blood chemistry (including anemia and leukopenia), skin rashes and drug dependence; and the surgical treatments are radical procedures that often profoundly alter the everyday life of the patient. Accordingly, there is a great need for treatments for IBD that are effective yet less severe in their side effects and are less invasive of the IBD sufferer's body and quality of life.

The search for the causes of IBD and more effective treatments has led several investigators to study diseased and normal tissue on a cellular level. This has led to observations of variations in the normal content of intestinal mucin (Podolsky, 1988 [1]) and to the observation of colonic glycoproteins that emerge only in diseased tissue (Podolsky and Fournier, 1988a [2], 1988b [3]). Researchers have observed that the cell adhesion molecule ICAM-1 is expressed at elevated levels in IBD tissue (Malizia et al., 1991 [4]). This molecule is thought to mediate leukocyte recruitment to sites of inflammation through adhesion to leukocyte surface ligands, i.e., LFA-1 (CD11a/CD18 complex) on all leukocytes and Mac-1 (CD11b/CD18) on phagocytes. (See, e.g., Springer, 1990 [5].) Because flareups of IBD are often accompanied by increased concentrations of neutrophils and lymphocytes in the intestinal submucosa, blocking of interactions between endothelial cell receptors (such as ICAM-1) and their leukocyte ligands (such as LFA-1, Mac-1) has bean proposed as a treatment for IBD.

Another cell adhesion molecule, VCAM-1 (vascular cell adhesion molecule-1) is expressed on inflamed endothelium and has been shown to recognize the α₄β₁ integrin, VLA-4, expressed on the surface of all leukocytes except neutrophils (See, e.g., Springer, 1990 [5]; WO 92/00751 (Nielsen et al. [33]); and Weller at al., 1991 [34]). VCAM-1 also has been found to be expressed constitutively in noninflamed tissue, including Peyer's patch follicular dendritic cells (Freedman et al., 1990 [6]; Rice et al., 1991 [7]). Additionally, besides mediating cell adhesion events, VCAM-1 also has recently been determined to play a costimulatory role, through VLA-4, in T cell activation (Burkly et al., 1991 [8]; Damle and Arrufo, 1991 [9]; van Seventer et al., 1991 [10]). Accordingly, further study of VCAM-1 has been taken up to investigate whether it plays a role as a regulator of the immune response as well as a mediator of adhesion in vivo.

PCT/WO 93/13798 published on 22 July 1993 describes a method for the treatment of asthma by administration of antibody, polypeptide or other molecule recognizing VLA-4. The document falls for consideration under Article 54(3) EPC.

It has now been surprisingly discovered that administering anti-VLA-4 antibody significantly reduces acute inflammation in a primate model for IBD. Cotton top tamarins suffering from a spontaneous intestinal inflammation comparable to ulcerative colitis in humans that were treated with an anti-VLA-4 antibody (HP1/2) showed significant reduction in inflammation of biopsied intestinal tissue.

### SUMMARY OF THE INVENTION

Accordingly, the present invention is based on novel methods for the treatment of IBD and provides new pharmaceutical compositions useful in the treatment of IBD. In particular, the present invention provides the manufacture of a medicament for use in a method comprising the step of administering to an IBD sufferer an anti-VLA-4 antibody, such as antibody HP1/2. Also contemplated is the use of analogous antibodies, antibody fragments, soluble proteins and small molecules that mimic the action of anti-VLA-4 antibodies in the treatment of IBD.

### DETAILED DESCRIPTION OF THE INVENTION

The technology for producing monoclonal antibodies is well known. Briefly, an immortal cell line (typically myeloma cells) is fused to lymphocytes (typically splenocytes) from a mammal immunized with whole cells expressing a given antigen, e.g., VLA-4, and the culture supernatants of the resulting hybridoma cells are screened for antibodies against the antigen. (See, generally, Kohler and Milstein, 1975 [11].)

Immunization may be accomplished using standard procedures. The unit dose and immunization regimen depend on the species of mammal immunized, its immune status, the body weight of the mammal, etc. Typically, the immunized mammals are bled and the serum from each blood sample is assayed for particular antibodies using appropriate screening assays. For example, anti-VLA-4 antibodies may be identified by immunoprecipitation of ¹²⁵I-labeled cell lysates from VLA-4-expressing cells. (See, Sanchez-Madrid et al., 1986 [13] and Hemler et al., 1987 [14].) Anti-VLA-4 antibodies nay also be identified by flow cytometry, e.g., by measuring fluorescent staining of Ramos cells incubated with an antibody believed to recognize VLA-4 (see, Elices et al., 1990 [15]). The lymphocytes used in the production of hybridoma cells typically are isolated from immunized mammals whose sera have already tested positive for the presence of anti-VLA-4 antibodies using such screening assays.

Typically, the immortal cell line (e.g., a myeloma cell line) is derived from the same mammalian species as the lymphocytes. Preferred immortal cell lines are mouse myeloma cell lines that are sensitive to culture medium containing hypoxanthine, aminopterin and thymidine ("HAT medium"). HAT-sensitive mouse myeloma cells may be fused to mouse splenocytes, e.g., using 1500 molecular weight polyethylene glycol ("PEG 1500"). Hybridoma cells resulting from the fusion are then selected using HAT medium, which kills unfused and unproductively fused myeloma cells (unfused splenocytes die after several days because they are not transformed). Hybridomas producing a desired antibody are detected by screening the hybridoma culture supernatants. For example, hybridomas prepared to produce anti-VLA-4 antibodies may be screened by testing the hybridoma culture supernatant for secreted antibodies having the ability to bind to a recombinant α₄-subunit-expressing cell line, such as transfected K-562 cells (see, Elices et al., [15]).

To produce anti VLA-4-antibodies, hybridoma cells that test positive in such screening assays may be cultured in a nutrient medium under conditions and for a time sufficient to allow the hybridoma cells to secrete the monoclonal antibodies into the culture medium. Tissue culture techniques and culture media suitable for hybridoma cells are well known. The conditioned hybridoma culture supernatant may be collected and the anti-VLA-4 antibodies optionally further purified by well-known methods.

Alternatively, the desired antibody may be produced by injecting the hybridoma cells into the peritoneal cavity of a mouse primed with 2,6,10,14-tetramethylpentadecane (PRISTANE; Sigma Chemical Co., St. Louis MO). The hybridoma cells proliferate in the peritoneal cavity, secreting the antibody, which accumulates as ascites fluid. The antibody may be harvested by withdrawing the ascites fluid from the peritoneal cavity with a syringe.

Several anti-VLA-4 monoclonal antibodies have been previously described (see, e.g., Sanchez-Madrid et al., 1986 [12]; Hemler et al. (1987) [13]; Pulido et al. (1991) [14]). For the experiments herein, an anti-VLA-4 monoclonal antibody designated HP1/2 (obtained from Biogen, Inc., Cambridge, MA) was used. The variable regions of the heavy and light chains of the anti-VLA-4 antibody HP1/2 have been cloned, sequenced and expressed in combination with constant regions of human immunoglobulin heavy and light chains. Such a chimeric HP1/2 antibody is similar in specificity and potency to the murine HP1/2 antibody, and may be useful in methods of treatment according to the present invention. Similarly, humanized recombinant anti-VLA-4 antibodies may be useful in these methods. The HP1/2 V_{H} DNA sequence and its translated amino acid sequences are set forth in SEQ ID NO: 1 and SEQ ID NO: 2, respectively. The HP1/2 V_{K} DNA sequence and its translated amino acid sequence are set forth in SEQ ID NO: 3 and SEQ ID NO: 4, respectively.

Monoclonal antibodies such as HP1/2 and other anti-VLA-4 antibodies (e.g., Mab HP2/1, HP2/4, L25, P4C2) capable of recognizing the α chain of VLA-4 will be useful in the present invention. It is most preferred that the antibodies will recognize the B1 or B2 epitopes of the VLA-α₄ chain (see, Pulido et al. (1991) [15]). While not wishing to be bound by one scientific theory, anti-VLA-4 antibodies used according to the method of the present invention may specifically inhibit, at least for an initial period, the migration of VLA-4-expressing leukocytes to inflamed sections of the gut. Or, the release of inflammatory mediators and cytokines by leukocytes already recruited to IBD tissue may be blocked by anti-VLA-4 antibodies that prevent some form of VCAM-1-mediated signal transduction, such as the T cell co-activation observed previously (e.g, Burkly et al. 1991 [8]). Monoclonal antibody HP1/2 has been shown to block leukocyte adhesion to VCAM-1-expressing cells but not to promote VLA-4-mediated T cell activation.

The method of the present invention comprises administering to a mammal suffering from inflammatory bowel disease a composition comprising an anti-VLA-4 antibody. The examples below set forth the results observed in cotton top tamarins. The physiological and histochemical similarities between a spontaneous chronic diffuse colitis observed in the cotton top tamarin (CTT) and IBD humans has been documented (see, e.g., Podolsky et al., 1985a [16], Podolsky et al., 1985b [17]). Prior studies have also demonstrated parallel responses in CTTs to therapeutic compounds used in the management of the human IBD (see, e.g., Madara et al., 1985 [18]). Accordingly, the results reported herein will be relevant and applicable to, and the method claimed will be useful in any mammal, including humans, suffering from IBD.

The anti-VLA-4 antibody administered in accordance with the present invention may be administered prophylactically to a chronic IBD sufferer, to bring about or maintain remission of the disease; however, preferably the method of the present invention is used to treat acute flareups of the disease.

The anti-VLA-4 antibody can be administered in the form of a composition comprising an anti-VLA-4 antibody and a pharmaceutically acceptable carrier. Preferably, the composition will be in a form suitable for intravenous injection. For acute flareups of ulcerative colitis or Crohn's disease, dosages of from 0.05 mg/kg-patient/day to 5.0 mg/kg-patient/day (preferably from 0.5 mg/kg-patient/day to 2.0 mg/kg-patient/day) may be used, although higher or lower dosages may be indicated with consideration to the age, sensitivity, tolerance, and other characteristics of the patient, the acuteness of the flareup, the history and course of the disease, plasma level and half-life of the antibody employed and its affinity for its recognition site, and other similar factors routinely considered by an attending physician. For maintenance of remission from active disease, dosages from 0.05 mg/kg-patient/day to 5.0 mg/kg-patient/day (preferably from 0.5 mg/kg-patient/day to 2.0 mg/kg-patient/day) may be used, although higher or lower dosages may be indicated and employed with advantageous effects considering the age, sensitivity, tolerance, and other characteristics of the patient, the pattern of flareups, the history and course of the disease, the plasma level and half-life of the antibody employed and its affinity for its recognition site, and other similar factors routinely considered by an attending physician. Dosages may be adjusted, for example, to provide a particular plasma level of antibody, e.g., in the range of 5-30 µg/ml, more preferably 10-15 µg/ml, for murine antibodies, and to maintain that level, e.g., for a period of time (e.g., 1 week) or until clinical results are achieved (e.g., flareup subsides). Chimeric and humanized antibodies, which would be expected to be cleared more slowly, will require lower dosages to maintain an effective plasma level. Also, antibodies or fragments having high affinity for VLA-4 will need to be administered less frequently or in lower doses than antibodies or antibody fragments of lesser affinity.

Suitable pharmaceutical carriers include, e.g., sterile saline, physiological buffer solutions and the like. The pharmaceutical compositions may additionally be formulated to control the release of the active ingredients or prolong their presence in the patient's system. Numerous suitable drug delivery systems are known for this purpose and include, e.g., hydrogels, hydroxmethylcellulose, microcapsules, liposomes, microemulsions, microspheres, and the like. Phosphate buffered saline (PBS) is a preferred carrier for injectible compositions.

It will also be recognized that for the purposes of the present invention, antibodies capable of binding to the α₄ subunit of VLA-4 must be employed. It is preferred that monoclonal antibodies be used.

In addition to naturally produced antibodies, suitable recombinant antibodies capable of binding to VLA-4 may alternatively be used. Such recombinant antibodies include antibodies produced via recombinant DNA techniques, e.g., by transforming a host cell with a suitable expression vector containing DNA encoding the light and heavy immunoglobulin chains of the desired antibody, and recombinant chimeric antibodies, wherein some or all of the hinge and constant regions of the heavy and/or the light chain of the anti-VLA-4 antibody have been substituted with corresponding regions of an immunoglobulin light or heavy chain of a different species (i.e., preferably the same species as the IBD sufferer being treated, to minimize immune response to the administered antibody). (See, e.g., Jones et al., 1986 [19], Ward et al., 1989 [20], and U.S. Patent 4,816,397 (Boss et al.) [21]) Recombinant antibodies specifically contemplated herein include CDR-grafted antibodies or "humanized" antibodies, wherein the hypervariable regions of, e.g., murine antibodies are grafted onto framework regions of, e.g., a human antibody. (See, e.g., Riechmann et al., 1988 [22]; Man Sung Co et al., 1991 [23]; Brown, Jr., 1991 [24].)

Furthermore, VLA-4-binding fragments of anti-VLA-4 antibodies, such as Fab, Fab', F(ab')₂, and F(v) fragments; heavy chain monomers or dimers; light chain monomers or dimers; and dimers consisting of one heavy chain and one light chain are also contemplated herein. Such antibody fragments may be produced by chemical methods, e.g., by cleaving an intact antibody with a protease, such as pepsin or papain, or via recombinant DNA techniques, e.g., by using host cells transformed with truncated heavy and/or light chain genes. Heavy and light chain monomers may similarly be produced by treating an intact antibody with a reducing agent such as dithiothreitol or β-mercaptoethanol or by using host cells transformed with DNA encoding either the desired heavy chain or light chain or both.

As an alternative to hybridoma technology, antibody fragments having the desired anti-VLA-4 specificities may be isolated by phage cloning methods. (See, e.g., Clackson et al., 1991 [25].)

Also, from the foregoing discussion it will be apparent that other molecules which bind to VLA-4 with sufficient specificity to inhibit VLA-4/VCAM-1 interactions or to inhibit transduction of VCAM-1-mediated signaling will be effective in the treatment of IBD in the same manner as anti-VLA-4 antibodies. Small molecules that mimic the binding domain of a VLA-4 ligand and fit the receptor domain of VLA-4 may be employed. (See also, Devlin et al., 1990 [27], Scott and Smith, 1990 [28], and U.S. Patent 4,833,092 (Geysen) [29], all incorporated herein by reference.) The use of such VLA-4-binding polypeptides or molecules that effectively decrease inflammation in IBD tissue in treated subjects is contemplated herein as an alternative method for treatment of IBD.

It is also contemplated that anti-VLA-4 antibodies may be used in combination with other antibodies having a therapeutic effect on IBD. For instance, to the extent that the beneficial effects reported herein are due to the inhibition of leukocyte recruitment to endothelium, combinations of anti-VLA-4 antibodies with other antibodies that interfere with the adhesion between leukocyte antigens and endothelial cell receptor molecules may be advantageous. For example, in addition to the use of anti-VLA-4 antibodies in accordance with this invention, the use of anti-ELAM-1 antibodies, anti-VCAM-1 antibodies, anti-ICAM-1 antibodies, anti-CDX antibodies, anti-CD18 antibodies, and/or anti-LFA-1 antibodies may be advantageous.

When formulated in the appropriate vehicle, the pharmaceutical compositions contemplated herein may be administered by any suitable means such as orally, intraesophageally or intranasally, as well as subcutaneously, intramuscularly, intravenously, intraarterially, or parenterally. Ordinarily intravenous (i.v.) or parenteral administration will be preferred to treat flareup conditions; oral administration in a timed release vehicle will be preferred to maintain remission.

Improvement for IBD patients as a result of the methods of this invention can be evaluated by any of a number of methods known to practitioners in this art. For example, improvement in observed symptomology such as the Truelove-Witts criteria (see, e.g., Lichtiger, et al., 1990 [30]) may be used, or specimens of colon tissue may be biopsied and characterized histologically (see, e.g., Madara et al., 1985 [18]).

The methods and compositions of the present invention will be further illuminated by reference to the following examples, which are presented by way of illustration and not of limitation.

### EXAMPLE I

### VCAM1 Expression in the Colon

Experiments were performed to determine whether active IBD involved the expression of endothelial cell surface proteins involved in leukocyte adhesion. Expression of VCAM-1 in colon tissue of IBD sufferers and normal or uninvolved colon tissue controls was evaluated. Human colonoscopic biopsy tissue samples were obtained, with informed consent, and prepared as frozen sections by mounting in OCT compound (TissueTek) and quick freezing in isopentane/liquid nitrogen. The human colon samples were from normal colon, active ulcerative colitis colon (UC-active), inactive ulcerative colitis colon (UC-inactive), uninvolved ulcerative colitis colon (UC-uninvolved), active Crohn's Disease colon (CD-active), and uninvolved Crohn's Disease colon (CD-uninvolved).

Frozen sections (∼4µ) were placed on gelatin-coated slides (1% gelatin, heated at 60° C for 1-2 min., air dried, 1% formaldehyde-at room temp., air dried), air dried 30 minutes, fixed in acetone for ten minutes at 4° C, washed three times in PBS and treated with 0.3% H₂O₂ in methanol (30 min., room temp.). The slides were then washed with PBS for 30 minutes, incubated with dilute normal human serum (1:100), and incubated with anti-VCAM-1 antibody 4B9 (1:100; obtained as a gift from Dr. John Harlan) for 60 minutes at room temperature. Control slides were incubated with an anti-bovine serum albumin (anti-BSA) antibody (Sigma Chemical Co., St. Louis MO). The samples were then washed with PBS for 10 minutes and incubated with a secondary biotinylated rabbit anti-mouse immunoglobulin (Dako Corp., Santa Barbara, CA) for 60 minutes at room temperature, then visualized using avidin-linked peroxidase (VECTASTAIN, Vector Labs, Burlingame CA).

The results of these tests are set forth in the following TABLE I:

**TABLE I**

| Endothelial Cell Staining In Human Tissue | | |
|---|---|---|
| Tissue (n) | VCAM-1 Expression | |
| | n | (%) |
| Normal (11) | 6 | (54.4) |
| UC active (23) | 14 | (60.9) |
| UC inactive (8) | 5 | (62.5) |
| UC uninvolved (10) | 4 | (40.0) |
| CD active (9) | 5 | (55.5) |
| CD uninvolved (12) | 7 | (58.3) |

These data confirm the observations such as those reported by Freedman et al. [6] and Rice et al. [7] that VCAM-1 is expressed in both IBD-involved colon tissue and in normal colon tissue. In both CD and UC tissues, VCAM-1 was observed by immunocytochemistry in about 60% of samples.

### EXAMPLE II

### Anti-VLA-4 Antibody Recognition of CTT White Blood Cells

An anti-VLA-4 monoclonal antibody (HP1/2, obtained from Biogen, Inc., Cambridge MA) was tested to confirm that it recognized an epitope on CTT leukocytes.

Blood samples (3 ml) from CTTs were heparinized and the CTT peripheral blood mononuclear leukocytes (PBLs) were isolated using a Ficoll-Hypaque gradient (Pharmacia) according to the manufacturer's instructions for isolation of human PBLs. CTT PBLs were examined for their ability to bind to the murine anti-human VLA-4 monoclonal antibodies HP1/2 and HP2/1 by FACS analysis using a Becton Dickenson FACStar and standard techniques (see, e.g., Lobb et al., 1991a [31]). Both monoclonal antibodies bound to CTT PBLs, indicating that both human and CTT VLA4 have similar epitopes recognized by these two antibodies.

CTT PBLs were also observed to adhere to microtiter plates coated with immobilized recombinant soluble human VCAM-1 (Biogen, Inc.), which binding was blocked by HP1/2 and HP2/1. These results show that CTT PBLs bind to VCAM-1 in a VLA-4-dependent manner, and that HP1/2 and HP2/1 block the interaction of CTT VLA-4 with human VCAM-1. (Cf. Lobb et al., 1991b [32].)

### EXAMPLE III

### Cotton Ton Tamarin Trials

A stock solution in sterile saline of the anti-VLA-4 antibody, HP1/2 (IgG1), and a placebo control (saline only), were prepared for administration to ten cotton top tamarins (CTTs) exhibiting symptoms of spontaneous colitis (i.e., diarrhea, etc.; see, Madara et al. [18]). Five CTTs received HP1/2 and five received placebo, by intravenous injection. The CTTs receiving HP1/2 were injected with 1 mg HP1/2 per day (i.e., about 2 mg/kg/day, based on approximate half-kilogram weight of a CTT) for eight days (on Days 0, 1, 2, 3, 4, 5, 6, and 7 of the trial). Colon tissue samples obtained from the animals were biopsied every other day (on Days 0, 2, 4, 6, 8, and 10 of the trial).

Data from the biopsies were used to determine an acute inflammation index for each animal, giving a semi-quantitative analysis of the course of the colitis. (See, Madara et al. [18].) The inflammation indices before the trial began (Day 0) and at the end of the trial at Day 10 are set forth in Table II, below: ("Treated CTTs" received antibody HP1/2; "Control CTTs" received placebo)

**TABLE II**

| Treated CTTs | Day 0 | Day 10 |
|---|---|---|
| | AII* | AII |
| 1 | 2 | 0 |
| 2 | 1 | 0 |
| 3 | 1 | 0 |
| 4 | 2 | 0 |
| 5 | 2 | 1 |
| MEAN | 1.6 | 0.2 |

| Control CTTs | | |
|---|---|---|
| C1 | 2 | 0 |
| C2 | 2 | 1 |
| C3 | 1 | 1 |
| C4 | 2 | 2 |
| C5 | 2 | 2 |
| MEAN | 1.8 | 1.2 |

| | | |
|---|---|---|
| * AII = acute inflammation index | | |

These results show that treatment with anti-VLA-4 antibody resulted in a significant (p < 0.01) decrease in acute inflammation index.

### EXAMPLE IV

The trial described in Example III was repeated using 14 CTTs, 7 receiving HP1/2 and 7 receiving placebo. The change in acute inflammation index from Day 0 to Day 10 is set forth in Table III:

**TABLE III**

| Treated CTTs | Day 0 | Day 10 |
|---|---|---|
| | AII | AII |
| 6 | 2 | 0 |
| 7 | 2 | 0 |
| 8 | 2 | 0 |
| 9 | 2 | 0 |
| 10 | 2 | 0 |
| 11 | 2 | 1 |
| 12 | 2 | 2 |
| MEAN | 2.0 | 0.43 |

| Control CTTs | | |
|---|---|---|
| C6 | 2 | 2 |
| C7 | 2 | 2 |
| C8 | 1 | 1 |
| C9 | 2 | 1 |
| C10 | 2 | 1 |
| C11 | 2 | 0 |
| C12 | 1 | 0 |
| MEAN | 1.71 | 1.00 |

The foregoing results show a significant decrease in acute inflammation in the CTTs receiving HP1/2.

The foregoing examples are intended as an illustration of the method of the present invention and are not presented as a limitation of the invention as claimed hereinafter. From the foregoing disclosure, numerous modifications and additional embodiments of the invention will be apparent to those experienced in this art. For example, actual dosage used, the type of antibody, antibody fragment or analog used, mode of administration, exact composition, time and manner of administration of the treatment, and many other features all may be varied without departing from the above description.

### CITED PUBLICATIONS

[1] D. Podolsky, "Colonic Glycoproteins in Ulcerative Colitis: Potential Meaning in Heterogeneity," Inflammatory Bowel Diseases: Basic Research and Clinical Implications, Falk Symposium, Titisee, Germany, June 7-9, 1987 (Kluwer Academic Publishers; Boston 1987) pp. 449-56.
[2] D. Podolsky and D. Fournier, "Alterations in Mucosal Content of Colonic Glycoconjugates in Inflammatory Bowel Disease Defined by Monoclonal Antibodies," Gastroenterology, 95, pp. 379-87 (1988).
[3] D. Podolsky and D. Fournier, "Emergence of Antigenic Glycoprotein Structures in Ulcerative Colitis Detected Through Monoclonal Antibodies," Gastroenterology, 95, pp. 371-8 (1988).
[4] G. Malizia et al., "Expression of Leukocyte Adhesion Molecules by Mucosal Mononuclear Phagocytes in Inflammatory Bowel Disease," Gastroenterology, 100, pp. 150-9 (1991).
[5] T. Springer, "Adhesion Receptors of the Immune System," Nature, 346, pp. 425-34 (August 1990).
[6] A. Freedman et al., "Adhesion of Human B Cells to Germinal Centers in Vitro Involves VLA-4 and INCAM-110," Science, 249, pp. 1030-33 (1990).
[7] G. E. Rice et al., "Vascular and Nonvascular Expression of INCAM-110," Amer. J. Pathology, 138(2), pp. 385-93 (1991).
[8] L. Burkly, et al., "Signaling by Vascular Cell Adhesion Molecule-1 (VCAM-1) Through VLA-4 Promotes CD3-dependent T Cell Proliferation," Eur. J. Immunol., 21, pp. 2871-75 (1991).
[9] N. Damle and A. Aruffo, "Vascular Cell Adhesion Molecule 1 Induces T-cell Antigen Receptor-dependent Activation of CD4⁺ T Lymphocytes," Proc. Natl. Acad. Sci. USA, 88, pp. 6403-7 (1991).
[10] G. van Seventer et al., "Analysis of T Cell Stimulation by Superantigen Plus Major Histocompatibility Complex Class II Molecules or by CD3 Monoclonal Antibody: Costimulation by Purified Adhesion Ligands VCAM-1, ICAM-1, but Not ELAM-1," J. Exp. Medicine, 174, pp. 901-13 (1991).
[11] Kohler and Milstein, "Continuous Cultures of Fused Cells Secreting Antibody of Predefined Specificity," Nature, 256, pp. 495-7 (1975).
[12] F. Sanchez-Madrid et al., "VLA-3: A novel polypeptide association within the VLA molecular complex: cell distribution and biochemical characterization," Eur. J. Immunol., 16, pp. 1343-9 (1986).
[13] M. E. Hemler et al., "Characterization of the Cell Surface Heterodimer VLA-4 and Related Peptides," J. Biol. Chem., 262(24), pp. 11478-85 (1987).
[14] M. Elices et al., "VCAM-1 on Activated Endothelium Interacts with the Leukocyte Integrin VLA-4 at a Site Distinct from the VLA-4/Fibronectin Binding Site," Cell, 60, pp. 577-84 (1990).
[15] R. Pulido et al., "Functional Evidence for Three Distinct and Independently Inhibitable Adhesion Activities Mediated by the Human Integrin VLA-4," J. Biol. Chem., 266(16), pp. 10241-5 (1991).
[16] D. Podolsky, et al., "Colonic Mucin Composition in Primates Selective Alterations Associated with Spontaneous Colitis in the Cotton-top Tamarin," Gastroenterology, 88, pp. 20-5 (1985).
[17] D. Podolsky et al., "Spontaneous Colitis In Cotton-Top Tamarins: Histologic, Clinical and Biochemical Features of an Animal Model of Chronic Colitis," Digestive Diseases and Sciences, 30(4), Abstract, p. 396 (1985).
[18] J. Madara et al., "Characterization of Spontaneous Colitis in Cotton-Top Tamarin (*Saguinus oedipus*) and Its Response to Sulfasalazine," Gastroenterology, 88, pp. 13-19 (1985).
[19] P. Jones et al., "Replacing the Complementarity-Determining Regions in a Human Antibody with Those From a Mouse," Nature, 321, pp. 522-25 (1986).
[20] E. Ward et al., "Binding Activities of a Repertoire of Single Immunoglobulin Variable Domains Secreted From Escherichia coli," Nature, 341, pp. 544-6 (1989).
[21] U.S. Patent 4,816,397, Boss et al., "Multichain Polypeptides Or Proteins And Processes For Their Production", issued March 28, 1989.
[22] L. Riechmann et al., "Reshaping Human Antibodies for Therapy," Nature, 332, pp. 323-7 (1988).
[23] Man Sun Co et al., "Humanized Antibodies for Antiviral Therapy," Proc. Natl. Acad. Sci. USA, 88, pp. 2869-73 (1990).
[24] P. Brown, Jr. et al., "Anti-Tac-H, a Humanized Antibody to the Interleukin 2 Receptor, Prolongs Primate Cardiac Allograft Survival," Proc. Natl. Acad. Sci. USA, 88, pp. 2663-7 (1990).
[25] T. Clackson et al., "Making Antibody Fragments Using Phage Display Libraries," Nature, 352, pp. 624-28 (1991).
[26] L. Osborn et al., "Direct Expression Cloning of Vascular Cell Adhesion Molecule 1, a Cytokine-induced Endothelial Protein That Binds to Lymphocytes," Cell, 59, pp. 1203-11 (1989).
[27] J. Devlin et al., "Random Peptide Libraries: A Source of Specific Protein Binding Molecules," Science, 249, pp. 400-406 (1990).
[28] J. Scott and G. Smith, "Searching for Peptide Ligands with an Epitope Library," Science, 249, pp. 386-90 (1990).
[29] U.S. Patent 4,833,092, Geysen, "Method For Determining Mimotopes", issued May 23, 1989.
[30] S. Lichtiger and D. Present, "Preliminary Report: Cyclosporin in Treatment of Severe Active Ulcerative Colitis," Lancet, 336, pp. 16-19 (1990).
[31] R. Lobb et al., "Expression and Functional Characterization of a Soluble Form of Endothelial-Leukocyte Adhesion Molecule 1," J. Immunol., 147(1), pp. 124-29 (1991).
[32] R. Lobb et al., "Expression and Functional Characterization of a Soluble Form of Vascular Cell Adhesion Molecule 1," Biochem. Biophys. Res. Commun., 178(3), pp. 1498-1504 (1991).
[33] WO 92/00751, Nielsen et al., "A pharmaceutical Composition comprising a Cell Adhesion Molecule," published January 23, 1992.
[34] P. Weller et al., "Human Eosinophil Adherence to Vascular Endothelium Mediated by Binding to Vascular Cell Adhesion Molecule 1 and Endothelial Leukocyte Adhesion Molecule 1," Prog. Natl. Acad. Sci. USA, 88, pp. 7430-7433 (1991).

## Claims

1. Use of an anti-VLA-4 antibody for manufacture of a medicament for the treatment of inflammatory bowel disease which treatment comprises administering to a mammal suffering from inflammatory bowel disease a composition comprising an anti-VLA-4 antibody.

2. Use according to Claim 1, wherein the anti-VLA-4 antibody composition is administered intravenously.

3. Use according to Claim 1, wherein the anti-VLA-4 antibody is selected from the group consisting to HP1/2, HP2/1, HP2/4, L25, and P4C2.

4. Use according to Claim 1, wherein the anti-VLA-4 antibody is HP1/2, or a fragment thereof capable of binding to VLA-4.

5. Use according to Claim 1, wherein the composition is administered at a dosage so as to provide from 0.05 to 5.0 mg/kg of antibody, based on the weight of the inflammatory bowel disease sufferer.

6. Use according to Claim 1, wherein the composition is administered at a dosage so as to provide 0.5 to 2.0 mg/kg of antibody, based on the weight of the inflammatory bowel disease sufferer.

7. Use according to Claim 1, wherein the composition is administered in an amount effective to provide a plasma level of antibody in the mammal of 10-15 µg/ml.

8. Use according to Claim 1, wherein the mammal is a human.

9. Use according to Claim 8, wherein the mammal suffers from ulcerative colitis.

10. Use according to Claim 8, wherein the mammal suffers from Crohn's disease.

11. Use according to Claim 1, wherein the composition is administered during an acute flareup of the inflammatory bowel disease.

12. Use of an antibody, a recombinant antibody, a chimeric antibody, or fragments of such antibodies, capable of binding to the α₄ subunit of VLA-4, or a small molecule that binds to the VCAM-1-binding domain of VLA-4, or combinations of any of the foregoing, for manufacture of a medicament for the treatment of inflammatory bowel disease which treatment comprises administering to a mammal suffering from inflammatory bowel disease the medicament in an amount effective to provide relief to said mammal.

13. Use according to Claim 12, wherein the antibody, fragment or molecule is selected from monoclonal antibody HP1/2; Fab, Fab', F(ab')₂ or F(v) fragments of such antibody, or a small molecule that binds to the VCAM-1-binding domain of VLA-4.

14. Use according to Claim 12, wherein the medicament comprises a plurality of anti-VLA-4-binding fragments thereof.

15. Use according to Claim 12, wherein the medicament includes, in addition to anti-VLA-4, an anti-ELAM-1 antibody, an anti-ICAM-1 antibody, an anti-VCAM-1 antibody, an anti-CDX antibody, an anti-LFA-1 antibody, an anti-CD18 antibody or combinations of any such remedies.

16. Use according to Claim 12, wherein the anti-VLA-4 antibody is HP1/2, or a fragment thereof capable of binding to VLA-4.

17. Use according to Claim 12, wherein the medicament is administered at a dosage so as to provide from 0.05 to 5.0 mg/kg of antibody, antibody fragment or small molecule, based on the weight of the inflammatory bowel sufferer.

18. Use according to Claim 17, wherein the medicament is administered at a dosage so as to provide 0.5 to 2.0 mg/kg of antibody, antibody fragment polypeptide or small molecule, based on the weight of the inflammatory bowel sufferer.

19. Use according to Claim 12, wherein the medicament is administered in an amount effective to provide a plasma level of antibody in the mammal of 10-15 µg/ml.

20. Use of a monoclonal antibody recognising VLA-4 and a pharamceutically acceptable carrier for the manufacture of a pharmaceutical composition for the treatment of inflammatory bowel disease consisting essentially of the monoclonal antibody recognizing VLA-4 in the pharmaceutically acceptable carrier.

21. Use of an anti-VLA-4 antibody HP1/2 or a fragment thereof capable of binding to VLA-4 for the manufacture of a medicament for the treatment of inflammatory bowel disease which treatment comprises administering to a mammal suffering from inflammatory bowel disease a comprising anti-VLA-4 antibody HP1/2 or a fragment thereof capable of binding to VLA-4.

22. Use of a small molecule capable of binding to the VCAM-1-binding domain of VLA-4 for the manufacture of a medicament for the treatment of inflammatory bowel disease which treatment comprises administering to a mammal suffering from inflammatory bowel disease a small molecule capable of binding to the VCAM-1-binding domain of VLA-4.

## Patentansprüche

1. Verwendung eines Anti-VLA-4-Antikörpers zur Herstellung eines Medikaments für die Behandlung einer entzündlichen Darmerkrankung, welche Behandlung die Verabreichung einer einen Anti-VLA-4-Antikörper umfassenden Zusammensetzung an ein Säugetier, das an einer entzündlichen Darmerkrankung leidet, aufweist.

2. Verwendung nach Anspruch 1, wobei die Anti-VLA-4-Antikörper-Zusammensetzung intravenös verabreicht wird.

3. Verwendung nach Anspruch 1, wobei der Anti-VLA-4-Antikörper aus der Gruppe ausgewählt wird, die aus HP1/2, HP2/1, HP2/4, L25 und P4C2 besteht.

4. Verwendung nach Anspruch 1, wobei der Anti-VLA-4-Antikörper HP1/2 oder ein Fragment davon, das imstande ist, sich an VLA-4 zu binden, ist.

5. Verwendung nach Anspruch 1, wobei die Zusammensetzung in einer derartigen Dosierung verabreicht wird, daß 0,05 bis 5,0 mg/kg Antikörper, bezogen auf das Gewicht des an der entzündlichen Darmerkrankung Leidenden, bereitgestellt werden.

6. Verwendung nach Anspruch 1, wobei die Zusammensetzung in einer derartigen Dosierung verabreicht wird, daß 0,5 bis 2,0 mg/kg Antikörper, bezogen auf das Gewicht des an der entzündlichen Darmerkrankung Leidenden, bereitgestellt werden.

7. Verwendung nach Anspruch 1, wobei die Zusammensetzung in einer Menge verabreicht wird, die wirksam einen Gehalt des Antikörpers im Plasma des Säugetiers von 10-15 µg/ml schafft.

8. Verwendung nach Anspruch 1, wobei das Säugetier ein Mensch ist.

9. Verwendung nach Anspruch 8, wobei das Säugetier an Colitis ulcerosa leidet.

10. Verwendung nach Anspruch 8, wobei das Säugetier an Morbus Crohn leidet.

11. Verwendung nach Anspruch 1, wobei die Zusammensetzung während eines akuten Aufflackerns der entzündlichen Darmerkrankung verabreicht wird.

12. Verwendung eines Antikörpers, eines rekombinanten Antikörpers, eines chimären Antikörpers oder von Fragmenten derartiger Antikörper, die imstande sind, sich an die α4-Untereinheit von VLA-4 zu binden, oder eines kleinen Moleküls, das sich an den VCAM-1-Bindungsbereich von VLA-4 bindet, oder an Kombinationen der vorhergehenden zur Herstellung eines Medikaments für die Behandlung einer entzündlichen Darmerkrankung, welche Behandlung die Verabreichung des Medikaments an ein an einer entzündlichen Darmerkrankung leidendes Säugetier in einer Menge, die wirksam Linderung für das Säugetier schafft, aufweist.

13. Verwendung nach Anspruch 12, wobei der Antikörper, das Fragment oder Molekül aus dem monoklonalen Antikörper HP1/2; den Fab-, Fab'-, F(ab')₂- oder F(v)-Fragmenten eines derartigen Antikörpers; oder einem kleinen Molekül, das sich an den VCAM-1-Bindungsbereich von VLA-4 bindet, ausgewählt wird.

14. Verwendung nach Anspruch 12, wobei das Medikament eine Vielzahl von Anti-VLA-4-Bindungsfragmenten davon umfaßt.

15. Verwendung nach Anspruch 12, wobei das Medikament zusätzlich zu dem Anti-VLA-4- einen Anti-ELAM-1-Antikörper, einen Anti-ICAM-1-Antikörper, einen Anti-VCAM-1-Antikörper, einen Anti-CDX-Antikörper, einen Anti-LFA-1-Antikörper, einen Anti-CD18-Antikörper oder Kombinationen derartiger Heilmittel einschließt.

16. Verwendung nach Anspruch 12, wobei der Anti-VLA-4-Antikörper HP1/2 oder ein Fragment davon, das imstande ist, sich an VLA-4 zu binden, ist.

17. Verwendung nach Anspruch 12, wobei das Medikament in einer derartigen Dosierung verabreicht wird, daß 0,05 bis 5,0 mg/kg Antikörper, Antikörperfragment oder kleines Molekül, bezogen auf das Gewicht des an der entzündlichen Darmerkrankung Leidenden, bereitgestellt werden.

18. Verwendung nach Anspruch 17, wobei das Medikament in einer derartigen Dosierung verabreicht wird, daß 0,5 bis 2,0 mg/kg Antikörper, Antikörperfragment-Polypeptid oder kleines Molekül, bezogen auf das Gewicht des an der entzündlichen Darmerkrankung Leidenden, bereitgestellt werden.

19. Verwendung nach Anspruch 12, wobei das Medikament in einer Menge verabreicht wird, die wirksam einen Gehalt des Antikörpers im Plasma des Säugetiers von 10-15 µg/ml schafft.

20. Verwendung eines monoklonalen Antikörpers, der VLA-4 erkennt, und eines pharmazeutisch verträglichen Trägers zur Herstellung eines Pharmazeutikums für die Behandlung einer entzündlichen Darmerkrankung, das im wesentlichen aus dem monoklonalen Antikörper, der VLA-4 erkennt, in dem pharmazeutisch verträglichen Träger besteht.

21. Verwendung eines Anti-VLA-4-Antikörpers HP1/2 oder eines Fragments davon, das imstande ist, sich an VLA-4 zu binden, zur Herstellung eines Medikaments für die Behandlung einer entzündlichen Darmerkrankung, welche Behandlung die Verabreichung eines einen Anti-VLA-4-Antikörper umfassenden HP1/2 oder eines Fragments davon, das imstande ist, sich an VLA-4 zu binden, an ein an einer entzündlichen Darmerkrankung leidendes Säugetier, aufweist.

22. Verwendung eines kleinen Moleküls das imstande ist, sich an den VCAM-1-Bindungsbereich von VLA-4 zu binden, zur Herstellung eines Medikaments für die Behandlung einer entzündlichen Darmerkrankung, welche Behandlung die Verabreichung eines kleinen Moleküls das imstande ist, sich an den VCAM-1-Bindungsbereich von VLA-4 zu binden, an ein an einer entzündlichen Darmerkrankung leidendes Säugetier, aufweist.

## Revendications

1. Utilisation d'anti-corps anti-VLA-4 pour la fabrication d'un médicament pour le traitement d'une maladie intestinale inflammatoire, lequel traitement comprend l'administration d'une composition comprenant un anti-corps anti-VLA-4 à un mammifère souffrant de maladie intestinale inflammatoire.

2. Utilisation selon la revendication 1, dans laquelle la composition à base d'anti-corps anti-VLA-4 est administrée par vole intra-veineuse.

3. Utilisation selon la revendication 1, dans laquelle l'anti-corps anti-VLA-4 est choisi parmi le groupe constitué de HP1/2, HP2/1, HP2/4, L25 et P4C2.

4. Utilisation selon la revendication 1, dans laquelle l'anti-corps anti-VLA-4 est HP1/2 ou un fragment de celui-ci capable de se lier à VLA-4.

5. Utilisation selon la revendication 1, dans laquelle la composition est administrée suivant une posologie permettant de pourvoir à de 0,05 à 5,0 mg/kg d'anti-corps, sur la base du poids du sujet souffrant de maladie intestinale inflammatoire.

6. Utilisation selon la revendication 1, dans laquelle la composition est administrée suivant une posologie permettant de pourvoir à de 0,5 à 2,0 mg/kg d'anti-corps, sur la base du poids du sujet souffrant de maladie intestinale inflammatoire.

7. Utilisation selon la revendication 1, dans laquelle la composition est administrée en une quantité efficace pour pourvoir à un taux d'anti-corps dans le plasma du mammifère de 10-15 µg/ml.

8. Utilisation selon la revendication 1, dans laquelle le mammifère est un humain.

9. Utilisation selon la revendication 8, dans laquelle le mammifère souffre de colite ulcéreuse.

10. Utilisation selon la revendication 8, dans laquelle le mammifère souffre de la maladie de Crohn.

11. Utilisation selon la revendication 1, dans laquelle la composition est administrée lors d'une crise aiguë d'une maladie intestinale inflammatoire.

12. Utilisation d'un anti-corps, d'un anti-corps recombinant, d'un anti-corps chimérique, ou de fragments de tels anti-corps capables de se lier à la sous-unité α4 de VLA-4, ou d'une petite molécule qui se lie au domaine de liaison VCAM-1 de VLA-4, ou de combinaisons de n'importe lesquels des précédents, pour le fabrication d'un médicament pour le traitement d'une maladie intestinale inflammatoire, lequel traitement comprend l'administration du médicament à un mammifère souffrant de maladie intestinale inflammatoire en une quantité efficace pour procurer un soulagement au dit mammifère.

13. Utilisation selon la revendication 12, dans laquelle l'anti-corps, le fragment ou la molécule est choisi parmi un anti-corps monoclonal HP1/2; des fragments Fab, Fab', F(ab')₂ ou F(v) d'un tel anti-corps; ou d'une petite molécule qui se lie au domaine de liaison VCAM-1 de VLA-4.

14. Utilisation selon la revendication 12, dans laquelle le médicament comprend une pluralité de fragments se liant au anti-VLA-4.

15. Utilisation selon la revendication 12, dans laquelle le médicament comprend en plus de l'anti-corps anti-VLA-4, un anti-corps anti-ELAM-1, un anti-corps anti-ICAM-1, un anti-corps anti-VCAM-1, un anti-corps anti-CDX, un anti-corps anti-LFA-1, un anti-corps anti-CD18, ou des combinaisons de tels remèdes.

16. Utilisation selon la revendication 12, dans laquelle l'anti-corps anti-VLA-4 est HP1/2, ou un fragment de celui-ci capable de se lier au VLA-4.

17. Utilisation selon la revendication 12, dans laquelle le médicament est administré suivant une posologie permettant de pourvoir à de 0,05 à 5,0 mg/kg d'anti-corps, de fragment d'anti-corps ou de petite molécule sur la base du poids du sujet souffrant de maladie intestinale inflammatoire.

18. Utilisation selon la revendication 17, dans laquelle le médicament est administré suivant une posologie permettant de pourvoir à de 0,5 à 2,0 mg/kg d'anti-corps, de fragment polypeptide d'anti-corps ou de petite molécule sur la base du poids du sujet souffrant de maladie intestinale inflammatoire.

19. Utilisation selon la revendication 12, dans laquelle le médicament est administré en une quantité efficace pour pourvoir à un taux d'anti-corps dans le plasma du mammifère de 10-15 µg/ml.

20. Utilisation d'un anti-corps reconnaissant VLA-4 et d'un véhicule pharmaceutiquement acceptable pour la fabrication d'une composition pharmaceutique pour le traitement d'une maladie intestinale inflammatoire essentiellement constituée de l'anti-corps monoclonal reconnaissant VLA-4 dans le véhicule pharmaceutiquement acceptable.

21. Utilisation d'un anti-corps HP1/2 anti-VLA-4 ou d'un fragment de celui-ci capable de se lier au VLA-4 pour la fabrication d'un médicament pour le traitement d'une maladie intestinale inflammatoire, lequel traitement comprend l'administration un anti-corps HP1/2 anti-VLA-4 ou un fragment de celui-ci capable de se lier à VLA-4 à un mammifère souffrant de maladie intestinale inflammatoire.

22. Utilisation d'une petite molécule capable de se lier au domaine de liaison VCAM-1 de VLA-4 pour la fabrication d'un médicament pour le traitement d'une maladie intestinale inflammatoire, lequel traitement comprend l'administration d'une petite molécule capable de se lier au domaine de liaison VCAM-1 de VLA-4 à un mammifère souffrant de maladie intestinale inflammatoire.


SEQUENCE LISTING

(1) GENERAL INFORMATION:

(i) APPLICANT: Lobb, Roy R.

(ii) TITLE OF INVENTION: Treatment for Inflammatory Bowel Disease

(iii) NUMBER OF SEQUENCES: 4

(iv) CORRESPONDENCE ADDRESS:
(A) ADDRESSEE: Allegretti & Witcoff, Ltd.
(B) STREET: 10 South Wacker Drive, Suite 3000
(C) CITY: Chicago
(D) STATE: IL
(E) COUNTRY: US
(F) ZIP: 60606

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: PCT
(B) FILING DATE: 1 February 1993
(C) CLASSIFICATION:

(viii) ATTORNEY/AGENT INFORMATION:
(A) NAME: McNicholas, Janet M.
(B) REGISTRATION NUMBER: 32,918
(C) REFERENCE/DOCKET NUMBER: 92,308-A; D003 CIP PCT

(ix) TELECOMMUNICATION INFORMATION:
(A) TELEPHONE: 312-715-1000
(B) TELEFAX: 312-715-1234

(2) INFORMATION FOR SEQ ID NO:1:

(i) SEQUENCE CHARACTERISTICS:
(A) LENGTH: 360 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: misc_feature
(B) LOCATION: 1
(D) OTHER INFORMATION: /note= "pBAG159 insert: HP1/2 heavy
chain variable region; amino acid 1 is Glu (E) but Gln (Q)
may be substituted"

(ix) FEATURE:
(A) NAME/KEY: CDS
(B) LOCATION: 1..360

(xi) SEQUENCE DESCRIPTION: SEQ ID NO:1:

GTC AAA CTG CAG CAG TCT GGG GCA GAG CTT GTG AAG CCA GGG GCC TCA 48
Val Lys Leu Gln Gln Ser Gly Ala Glu Leu Val Lys Pro Gly Ala Ser
2 6 11 16

GTC AAG TTG TCC TGC ACA GCT TCT GGC TTC AAC ATT AAA GAC ACC TAT 96
Val Lys Leu Ser Cys Thr Ala Ser Gly Phe Asn Ile Lys Asp Thr Tyr
21 26 31

ATG CAC TGG GTG AAG CAG AGG CCT GAA CAG GGC CTG GAG TGG ATT GGA 144
Met His Trp Val Lys Gln Arg Pro Glu Gln Gly Leu Glu Trp Ile Gly
36 41 46

AGG ATT GAT CCT GCG AGT GGC GAT ACT AAA TAT GAC CCG AAG TTC CAG 192
Arg Ile Asp Pro Ala Ser Gly Asp Thr Lys Tyr Asp Pro Lys Phe Gln
51 56 61

GTC AAG GCC ACT ATT ACA GCG GAC ACG TCC TCC AAC ACA GCC TGG CTG 240
Val Lys Ala Thr Ile Thr Ala Asp Thr Ser Ser Asn Thr Ala Trp Leu
66 71 76 81

CAG CTC AGC AGC CTG ACA TCT GAG GAC ACT GCC GTC TAC TAC TGT GCA 288
Gln Leu Ser Ser Leu Thr Ser Glu Asp Thr Ala Val Tyr Tyr Cys Ala
86 91 96

GAC GGA ATG TGG GTA TCA ACG GGA TAT GCT CTG GAC TTC TGG GGC CAA 336
Asp Gly Met Trp Val Ser Thr Gly Tyr Ala Leu Asp Phe Trp Gly Gln
101 106 111

GGG ACC ACG GTC ACC GTC TCC TCA 360
Gly Thr Thr Val Thr Val Ser Ser
116 121

(2) INFORMATION FOR SEQ ID NO:2:

(i) SEQUENCE CHARACTERISTICS:
(A) LENGTH: 120 amino acids
(B) TYPE: amino acid
(D) TOPOLOGY: linear

(ii) MOLECULE TYPE: protein

(xi) SEQUENCE DESCRIPTION: SEQ ID NO:2:

Val Lys Leu Gln Gln Ser Gly Ala Glu Leu Val Lys Pro Gly Ala Ser
2 6 11 16

Val Lys Leu Ser Cys Thr Ala Ser Gly Phe Asn Ile Lys Asp Thr Tyr
21 26 31

Met His Trp Val Lys Gln Arg Pro Glu Gln Gly Leu Glu Trp Ile Gly
36 41 46

Arg Ile Asp Pro Ala Ser Gly Asp Thr Lys Tyr Asp Pro Lys Phe Gln
51 56 61

Val Lys Ala Thr Ile Thr Ala Asp Thr Ser Ser Asn Thr Ala Trp Leu
66 71 76 81

Gln Leu Ser Ser Leu Thr Ser Glu Asp Thr Ala Val Tyr Tyr Cys Ala
86 91 96

Asp Gly Met Trp Val Ser Thr Gly Tyr Ala Leu Asp Phe Trp Gly Gln
101 106 111

Gly Thr Thr Val Thr Val Ser Ser
116 121

(2) INFORMATION FOR SEQ ID NO:3:

(i) SEQUENCE CHARACTERISTICS:
(A) LENGTH: 318 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: CDS
(B) LOCATION: 1..318
(D) OTHER INFORMATION: /product= "HP1/2 light chain
variable region"

(ix) FEATURE:
(A) NAME/KEY: misc_feature
(B) LOCATION: 1
(D) OTHER INFORMATION: /note= "pBAG172 insert: HP1/2 light
chain variable region"

(xi) SEQUENCE DESCRIPTION: SEQ ID NO:3:

AGT ATT GTG ATG ACC CAG ACT CCC AAA TTC CTG CTT GTT TCA GCA GGA 48
Ser Ile Val Met Thr Gln Thr Pro Lys Phe Leu Leu Val Ser Ala Gly
1               5                   10                  15

GAC AGG GTT ACC ATA ACC TGC AAG GCC AGT CAG AGT GTG ACT AAT GAT 96
Asp Arg Val Thr Ile Thr Cys Lys Ala Ser Gln Ser Val Thr Asn Asp
            20                  25                  30

GTA GCT TGG TAC CAA CAG AAG CCA GGG CAG TCT CCT AAA CTG CTG ATA 144
Val Ala Trp Tyr Gln Gln Lys Pro Gly Gln Ser Pro Lys Leu Leu Ile
        35                  40                  45

TAT TAT GCA TCC AAT CGC TAC ACT GGA GTC CCT GAT CGC TTC ACT GGC 192
Tyr Tyr Ala Ser Asn Arg Tyr Thr Gly Val Pro Asp Arg Phe Thr Gly
    50                  55                  60

AGT GGA TAT GGG ACG GAT TTC ACT TTC ACC ATC AGC ACT GTG CAG GCT 240
Ser Gly Tyr Gly Thr Asp Phe Thr Phe Thr Ile Ser Thr Val Gln Ala
65                  70                  75                  80

GAA GAC CTG GCA GTT TAT TTC TGT CAG CAG GAT TAT AGC TCT CCG TAC 288
Glu Asp Leu Ala Val Tyr Phe Cys Gln Gln Asp Tyr Ser Ser Pro Tyr
                85                  90                  95

ACG TTC GGA GGG GGG ACC AAG CTG GAG ATC 318
Thr Phe Gly Gly Gly Thr Lys Leu Glu Ile
            100                 105

(2) INFORMATION FOR SEQ ID NO:4:

(i) SEQUENCE CHARACTERISTICS:
(A) LENGTH: 106 amino acids
(B) TYPE: amino acid
(D) TOPOLOGY: linear

(ii) MOLECULE TYPE: protein

(xi) SEQUENCE DESCRIPTION: SEQ ID NO:4:

Ser Ile Val Met Thr Gln Thr Pro Lys Phe Leu Leu Val Ser Ala Gly
1               5                   10                  15

Asp Arg Val Thr Ile Thr Cys Lys Ala Ser Gln Ser Val Thr Asn Asp
            20                  25                  30

Val Ala Trp Tyr Gln Gln Lys Pro Gly Gln Ser Pro Lys Leu Leu Ile
        35                  40                  45

Tyr Tyr Ala Ser Asn Arg Tyr Thr Gly Val Pro Asp Arg Phe Thr Gly
     50                  55                  60

Ser Gly Tyr Gly Thr Asp Phe Thr Phe Thr Ile Ser Thr Val Gln Ala
65                  70                  75                  80

Glu Asp Leu Ala Val Tyr Phe Cys Gln Gln Asp Tyr Ser Ser Pro Tyr
                85                  90                  95

Thr Phe Gly Gly Gly Thr Lys Leu Glu Ile
            100                 105